# EUROPEAN PATENT SPECIFICATION

(11) **EP 3 292 814 B1**
(45) Date of publication and mention of the grant of the patent: **07.08.2019**
(21) Application number: 17190067.3
(22) Date of filing: 08.09.2017
(51) Int. Cl.: A61B 5/04

(54) **ELECTROMEDICAL EQUIPMENT**
ELEKTROMEDIZINISCHES GERÄT
APPAREIL ÉLECTROMÉDICAL

(30) Priority: 09.09.2016 IT 201600091352
(43) Date of publication of application: 14.03.2018
(73) Proprietor: Medicaltech Srl, 38068 Rovereto (TN) (IT)
(72) Inventor: QUAINI, Giuseppe, 38068 ROVERETO (TN) (IT)
(74) Representative: Baroni, Matteo

(56) References cited:
- WO-A2-2009/112972
- US-A1- 2011 282 225
- US-A1- 2014 364 756

## Description

### [FIELD OF THE INVENTION]

The present invention relates to an electromedical equipment.

In particular, said electromedical equipment is dedicated to detecting cardiac signals for the execution of electrocardiograms or Holter tests (also referred to as "dynamic electrocardiograms").

### [PRIOR ART]

As is known, the conditions of proper operation of an individual's cardiac apparatus can be evaluated by means of different types of analysis; analyses can also be made by referring to electric signals generated by the heart during the contraction and relaxation movements of the ventricles.

A first verification that can be made is the so-called static electrocardiogram (ECG), wherein the action potentials of the myocardial fibres are recorded by means of suitable electrodes placed in contact with the skin of the patient at rest.

Electrodes are normally employed in order to be able to detect the projections of the electric axis of the heart in different planes; the recording may last a few tens of seconds and, when the detection phase is over, the graph is analyzed by the doctor, who will then formulate a diagnosis.

A second analysis which is normally carried out is the so-called dynamic electrocardiogram according to Holter (or simply "Holter"), wherein electrocardiographic signals are recorded for a period of time of, for example, 24 hours; said period of time may last, for example, up to 120 hours. Due to the great amount of data to be taken into consideration, as few as four electrodes may be used in this case for detecting some projections of the electric axis which are representative of the heart's behaviour.

In this case as well, at the end of the detection phase the whole graph must be examined by a qualified physician, who will then identify any recorded pathologies.

In light of the above, it is apparent that, especially in the case of Holter tests, the amount of data to be analyzed is considerable and the doctor's task is particularly difficult and time-consuming.

In order to remedy this problem at least partially, the prior art provides equipment known as "ECG Event Recorders": these are devices designed for recording the electrocardiographic signal when an actuation command is issued by the user.

In other words, an ECG Event Recorder is normally in a stand-by condition; when the patient feels symptoms indicating that his/her cardiac apparatus is not working properly, he/she can press a specific button (or another similar triggering tool) in order to start the recording of the cardiac signal.

The recordings relating to those periods of time when the patient perceived symptoms of discomfort are then supplied to the doctor, so that the corresponding graphs can be correctly interpreted.

It is plain that also this latter technical solutions suffers from apparent operational drawbacks. First of all, it cannot take into account any pathologies that do not cause the patient to feel any evident and clearly identifiable symptom.

In addition, even if the patient actuates the device when the symptom arises, it will not be possible to analyze the behaviour of the cardiac apparatus in the time interval immediately preceding the start of the recording; such an analysis may however be extremely useful to better comprehend the type of disorder that occurred.

Document US 2011/282225 A1 discloses a computing device including a memory device, a communication module, an interface, a processor, a display controller, and an input device. The communication module wirelessly receives a stream of first electrogram waveforms (EGMs) from an implantable medical device (IMD). The interface receives a stream of second EGMs. The processor stores the first and second EGMs and retrieves the stored first and second EGMs. The display controller displays the first and second EGMs together when the first and second EGMs are received. The input device is configured to receive a selection command from a user. The processor retrieves a portion of at least one of the first and second stored EGMs in response to the selection command. The display controller displays the retrieved portion of the at least one of the first and second stored EGMs while the processor concurrently stores the streams of the first and second EGMs.

### [OBJECTS AND SUMMARY OF THE INVENTION]

It is one object of the present invention to provide an equipment for detecting cardiac signals which can reliably detect cardiac signal portions that are significant, i.e. associated with the occurrence of actual pathologies in the patient.

It is another object of the present invention to provide an equipment that allows for an immediate comparative analysis between the occurrence of the symptoms in the patient and the actual disorders suffered by the cardiac apparatus.

It is a further object of the present invention to provide an equipment that can operate in a complete and reliable manner while using a limited amount of memory.

These and other objects are substantially achieved through an electromedical equipment as set out in the appended claims.

### [BRIEF DESCRIPTION OF THE DRAWINGS]

Further features and advantages will become more apparent in the light of the following detailed description of some preferred but non-limiting embodiments of the invention.

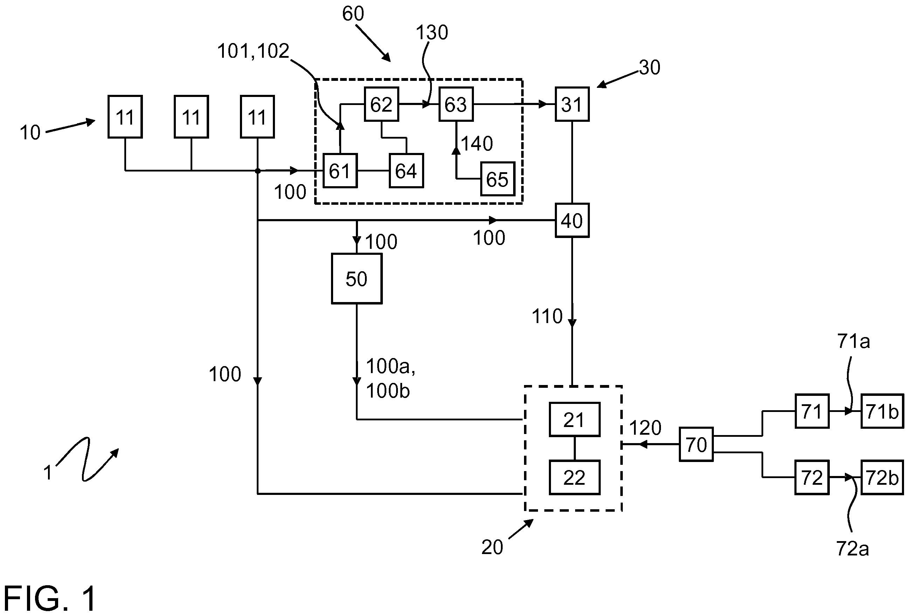

The following description will refer to the annexed Figure 1, also provided by way of non-limiting example.

### [DETAILED DESCRIPTION OF THE INVENTION]

With reference to the annexed drawings, numeral 1 designates as a whole an electromedical equipment according to the present invention.

The electromedical equipment 1 performs the task of detecting cardiac signals for recording electrocardiograms and/or Holter graphs.

The equipment 1 comprises, first of all, sensor means 10 for detecting a cardiac signal 100.

The sensor means 10 preferably comprise a plurality of electrodes 11, which are placed in contact with the patient's skin when the equipment 1 is in use.

The electrodes can conveniently be associated with suction cups or adhesive means (not shown in the drawing), in order to ensure that the electrodes will stay in position on the patient's body.

The sensor means 10 are positioned in such a way that they can detect signals representative of the electric potentials generated in the heart.

For "static" electrocardiograms, the electrodes are positioned on the chest (e.g. 6 electrodes) and on the limbs of the patient (one electrode per limb).

For Holter (or dynamic) electrocardiograms, the electrodes are positioned on the patient's chest (e.g. 4 electrodes).

Advantageously, the electrodes 11 of the equipment 1 are set in each one of the above-mentioned positions, so as to allow recording both a static electrocardiogram and a dynamic electrocardiogram.

For the purpose of storing the information thus acquired, the equipment 1 further comprises a storage unit 20; the latter is arranged to store, at least partially, the cardiac signal 100.

More in detail, the storage unit 20 is provided with a permanent memory 21, into which a preset number of portions of the cardiac signal 100 can be stored.

It should be noted that, in the present context, the term "permanent" means that the data stored in the memory 21 are preserved until an operator issues an explicit deletion command; in particular, the term "permanent" is used herein as opposite to the term "temporary", which, as will become apparent hereinafter, refers to a buffer in which the data are continuously overwritten, so that the oldest information is lost.

Storage of the cardiac signal 100 into the permanent memory 21 can be actuated as a function of the characteristics of the cardiac signal 100 itself and of main reference parameters 31 related to said cardiac signal.

In fact, the equipment 1 comprises an auxiliary memory 30 in which the main reference parameters 31 are stored; the main reference parameters 31 are representative of predefined cardiac pathologies that may arise in the patient.

Preferably, such parameters have been defined at international level by recognized bodies, so that the equipment 1 can meet the required quality and reliability standards.

The equipment 1 further comprises a comparator block 40 connected to the sensor means 10 and to the auxiliary memory 30; the comparator block 40 compares the cardiac signal 100 with the main reference parameters 31 and, depending on this comparison, it will generate, if necessary, an appropriate main command signal 110 in order to actuate the storage 20 of the cardiac signal 100 into the permanent memory 21.

In this way, as soon as an anomaly (i.e. one of the pathologies stored beforehand by means of the main reference parameters 31) is detected in the cardiac signal 100, the recording of a portion of the cardiac signal 100 will be initiated, so that it can subsequently be analyzed by a qualified physician.

As aforementioned, the main reference parameters 31 are representative of pathologies that may arise in the patient's cardiac apparatus.

In order to make the decision made by the comparator block 40 more reliable, it is necessary that the main reference parameters 31 have been properly calibrated in accordance with the general physical characteristics of the patient.

In fact, individuals of different sex, stature, weight, age, etc. will have different discrimination thresholds for the identification of cardiac pathologies.

Therefore, the equipment 1 further comprises a learning unit 60 provided with a learning registry 61, into which at least one initial portion 101 of the cardiac signal 100 is stored; in practice, when the equipment 1 starts operating, the first cardiac signal portion detected (e.g. the first 8 seconds) is stored into the learning registry 61.

Suitable processing means 62 then compute setting parameters 130 as a function of the characteristics of said initial portion 101; the setting parameters 130 are representative of the main characteristics of the cardiac signal 100 in a condition of proper operation of the patient's organism.

The learning unit 60 further comprises an auxiliary registry 65 storing generic parameters 140 representative of cardiac pathologies; such parameters 140 are generic and do not take into account the patient's specific characteristics.

The learning unit 60 further comprises a calculation block 63 for calculating the main reference parameters 31 as a function of said setting parameters 130; in particular, the calculation block 63 is connected to the processing means 62 and to the auxiliary registry 65 in order to be able to define the main reference parameters 31 as a function of the setting parameters 130 (i.e. the patient's characteristics) and also as a function of the generic parameters 140 (representing detectable pathologies).

The main reference parameters 31 thus calculated are then stored into the auxiliary memory 30.

Aiming at further improving the accuracy of the determination of the main reference parameters 31, the learning unit 60 further comprises updating means 64; the latter are connected to the learning registry 61 in order to actuate also the recording of auxiliary portions 102 of the cardiac signal 100.

Said auxiliary portions 102 are preferably acquired and stored in predefined time intervals, according to a preset frequency; by way of example, the auxiliary portions 102 may last about 30 seconds and be detected every 3 hours.

The updating means 64 are also connected to the processing means 62 in order to actuate the latter and update the setting parameters 130 as a function of the characteristics of the auxiliary portions 102 of the cardiac signal 100.

The calculation block 63 is then also actuated, so that the main reference parameters 31 can be determined by taking into account also the setting parameters 130 updated in accordance with the characteristics of the auxiliary portions 102.

In light of the above, it is clear that the reference parameters 31 can be optimally defined to make the operation of the comparator block 40 and of the entire equipment 1 as a whole more accurate and reliable.

It will now be described how the cardiac signal 100 is stored.

The equipment 1 comprises a temporary storage registry 50 connected to the sensor means 10 for temporarily storing portions of the cardiac signal 100 having a predefined time duration.

As will become apparent below, when said main command signal 110 is entered, the contents of the temporary registry 50 are transferred into the memory 21 for permanent storage.

In practice, the temporary registry 50 may be a "circular" buffer, i.e. a buffer wherein data are stored and overwritten sequentially with a predefined frequency.

It must be pointed out that the storage capacity of the temporary registry 50 is insufficient to contain the entire cardiac signal 100; for example, the cardiac signal 100 may last 24 hours (in the case of a Holter test), while the temporary registry 50 only has enough capacity to contain approx. 40 seconds of recording. Note that a Holter test may last as long as, for example, 120 hours.

The oldest data are thus replaced with the data just acquired; therefore, the last 40 seconds of cardiac signal recording will always be available.

Temporary storage of the cardiac signal 100 into the temporary registry 50 occurs as described above, regardless of the generation of the command signal 110; the latter is used for transferring, at least partially, the contents of the temporary registry 50 into the permanent memory 21.

In fact, when the comparator 40 actuates the storage unit 20 following the comparison between the cardiac signal 100 and the main reference parameters 31, the storage unit 20 will save first and second portions 100a, 100b of the cardiac signal 100 into its own permanent memory 21.

The second portion 100b is, in particular, that portion of the cardiac signal 100 which immediately precedes the instant at which the storage unit 20 is actuated; said second portion 100b is taken from the temporary registry 50, which, as aforementioned, keeps such data until they are overwritten.

For example, the second portion 100b may have a time duration of approx. 20 seconds.

On the other hand, the first portion 100a is that portion of the cardiac signal 100 which immediately follows the actuation of the storage unit 20; the first portion 100a can thus be stored into the permanent memory 21 in real time, since it comes directly from the sensor means 10 or goes through the temporary registry 50.

Also the first portion 100a may have, for example, a time duration of approx. 20 seconds; the result of the above-described procedure is that, following the generation of the main command signal 110, the permanent memory 21 can save a data set representative of the cardiac signal 100 in the 20 seconds before and in the 20 seconds after the detection of the anomaly.

Of course, the above-mentioned time durations are merely illustrative and may change according to specific requirements without departing from the general principles of this description.

Advantageously, the storage unit 20 is provided with a time storage block 22 for storing data representative of the time instants at which the cardiac signal 100 is detected and/or saved into said permanent memory 21.

Thus, the doctor who will have to analyze the recorded information will be able to know the time of day when the anomaly occurred, which caused the partial storage of the cardiac signal 100.

In the preferred embodiment, the equipment 1 further comprises a drive block 70, which can be actuated by the patient when he/she perceives discomfort that might be caused by a disorder in his/her cardiac apparatus.

In such a case, the drive block 70 will generate an auxiliary command signal 120 in order to actuate the storage unit 20 and cause the cardiac signal 100 to be partially saved as described with reference to the main command signal 100.

More in particular, also in case of user-initiated actuation, the permanent memory 21 will store first and second portions 100a, 100b of the cardiac signal 100, respectively related to the intervals immediately preceding and immediately following the reception of the auxiliary command signal 120 by the storage unit 20.

Furthermore, the time storage block 22 provides for storing data useful for identifying the instant when the auxiliary command signal 120 was generated, i.e. the instant at which the patient perceived the disorder.

In practice, the drive block 70 may consist of a push-button or an equivalent device capable of generating the auxiliary command signal 120.

The main command signal 110 or the auxiliary command signal 120 may sometimes be received by the storage unit 20 when a process of saving the cardiac signal 100 into the permanent memory is already in progress; typically this may happen when a given pathology is first detected by the equipment 1 and is shortly afterwards perceived also by the patient because of the resulting disorder.

In such a case, it is necessary to appropriately manage the overlapping of the two commands.

Moreover, different time durations may be used upon reception of the main command signal 110 and auxiliary command signal 120, i.e. a first time duration and a second time duration, different from each other.

Advantageously, the equipment 1 is provided with a microphone 71, which can be actuated via the drive block 70.

In this manner, the patient has the possibility of vocally inputting first information 71a about symptoms perceived when he/she actuated said drive block 70. In other words, after having actuated the drive block 70 (thereby signalling the presence of a generic disorder), the patient can input a vocal message (first information 71a) in order to provide details regarding the symptoms connected to that sensation of discomfort.

The Applicant believes that the use of the microphone 71 is particularly useful and advantageous, since it allows the patient to provide useful information in a simple and straightforward manner, even though he/she is in non-optimal health conditions.

Preferably, the first information 71a is stored into a first memory area 71b of the equipment 1, so that it can then be analyzed by qualified personnel.

Preferably, the equipment 1 further comprises a touch-screen display 72, through which the patient can input second information 72a about the symptoms perceived when he/she actuated the drive block 70.

In practice, following the actuation of the drive block 70, also the display 71 is turned on, so that the user can use an additional tool in order to input data representative of the perceived symptoms.

In particular, the display 72 is configured for presenting the patient with a plurality of options to choose from in order to input said second information 72a. The choice of one or more of the presented options is made by the patient directly on the display 72, due to the use of touch-screen technology.

Preferably, the second information 72a is stored into a second memory area 72b of the equipment 1.

The Applicant believes that the use of the touch-screen display 72 is particularly advantageous, in that it allows the user to input important information even when the patient is unable to speak, e.g. because of excessive weariness due to an ongoing disorder.

In light of the above, the equipment 1 is provided with an electronic processing system (e.g. a microprocessor system) capable of performing the above-described functions; in other words, said comparator block 40, storage unit 20 and learning unit 60 can be implemented as one suitably programmed CPU.

The division into blocks has been adopted herein merely to clarify the functions that are carried out by the equipment 1 according to the invention.

Likewise, the permanent memory 21, the time storage block 22, the auxiliary memory 30, the temporary registry 50, the auxiliary registry 65, the first memory area 71b and the second memory area 72b can be implemented as one or more electronic memories associated with the above-mentioned CPU.

The invention attains some important advantages.

First of all, the equipment according to the invention can reliably detect significant portions of the cardiac signal, i.e. portions associated with the onset of actual pathologies in the patient.

Another advantage emerges from the fact that the patient has the possibility to enter, in a simple, straightforward and reliable manner, useful information about the perceived sensations of discomfort, thus providing the medical personnel with data useful for identifying any pathologies.

A further advantage is due to the fact that, in order to operate correctly and fully, the equipment only requires a very small memory, since it only records important and significant information.

## Claims

1. Equipment for detecting cardiac signals, comprising:
a) sensor means (10) for detecting at least one cardiac signal (100) of a patient;
b) a storage unit (20) connected to said sensor means (10) to at least partially store said cardiac signal (100);
c) an auxiliary memory (30) containing one or more main reference parameters (31) for said cardiac signal (100);
d) a comparator block (40) to compare said cardiac signal (100) with said one or more main reference parameters (31) and actuate said storage unit (20) as a function of said comparison by means of a respective main command signal (110);
e) a drive block (70) that can be actuated by the patient in order to actuate said storage unit (20) by means of an auxiliary command signal (120);
f) a microphone (71), which can be actuated by means of said drive block (70) to allow said patient to vocally input first information (71a) relating to symptoms perceived upon the actuation of said drive block (70)
g) a touch-screen display (72), by means of which said patient can input second information (72a) relating to symptoms perceived upon the actuation of said drive block (70), wherein said display (72) is configured to present said patient with a plurality of options to choose from in order to input said second information (72a).

2. Equipment according to claim 1, wherein said first information (71a) is stored in a first memory area (71b) of said equipment (1).

3. Equipment according to claim 1, wherein the possibility of inputting said second information (72b) by means of said display (72) is activated by said drive block (70).

4. Equipment according to anyone of the preceding claims, wherein said second information (72a) is stored in a second memory area (72b) of said equipment (1).

5. Equipment according to any one of the preceding claims, further comprising a learning unit (60) provided with:
a) a learning registry (61) to store at least one initial portion (101) of said cardiac signal (100);
b) processing means (62) to calculate setting parameters (130) as a function of characteristics of said initial portion (101) of the cardiac signal (100).

6. Equipment according to claim 5, wherein said learning unit (60) further comprises a calculation block (63) to calculate said main reference parameters (31) as a function of said setting parameters (130).

## Patentansprüche

1. Vorrichtung zum Erfassen von Herzsignalen, umfassend:
a) Sensormittel (10) zum Erfassen mindestens eines Herzsignals (100) eines Patienten;
b) eine Speichereinheit (20), die mit den Sensormitteln (10) verbunden ist, um das Herzsignal (100) zumindest teilweise zu speichern;
c) einen Hilfsspeicher (30), der einen oder mehrere Hauptreferenzparameter (31) für das Herzsignal (100) enthält;
d) einen Vergleichsblock (40) zum Vergleichen des Herzsignals (100) mit dem einen oder den mehreren Hauptreferenzparametern (31) und zum Betätigen der Speichereinheit (20) in Abhängigkeit des Vergleichs mittels eines entsprechenden Hauptbefehlssignals (110);
e) einen Antriebsblock (70), der von dem Patienten betätigt werden kann, um die Speichereinheit (20) mittels eines Hilfsbefehlssignals (120) zu betätigen;
f) ein Mikrofon (71), das mittels des Antriebsblocks (70) betätigt werden kann, um es dem Patienten zu ermöglichen, erste Informationen (71a) bezüglich der beim Betätigen des Antriebsblocks (70) wahrgenommenen Symptome mündlich einzugeben.
g) eine Touchscreen-Anzeige (72), mittels derer der Patient zweite Informationen (72a) bezüglich der Symptome eingeben kann, die bei der Betätigung des Antriebsblocks (70) wahrgenommen werden, wobei die Anzeige (72) konfiguriert ist, um dem Patienten eine Vielzahl von Optionen zur Auswahl zu präsentieren, um die zweiten Informationen (72a) einzugeben.

2. Vorrichtung nach Anspruch 1, wobei die erste Information (71a) in einem ersten Speicherbereich (71b) der Vorrichtung (1) gespeichert ist.

3. Vorrichtung nach Anspruch 1, wobei die Möglichkeit der Eingabe der zweiten Information (72b) mittels der Anzeige (72) durch den Antriebsblock (70) aktiviert wird.

4. Vorrichtung nach einem der vorhergehenden Ansprüche, wobei die zweite Information (72a) in einem zweiten Speicherbereich (72b) der Vorrichtung (1) gespeichert ist.

5. Vorrichtung nach einem der vorstehenden Ansprüche, ferner umfassend eine Lerneinheit (60), die umfasst:
a) ein Lernregister (61), um mindestens einen Anfangsabschnitt (101) des Herzsignals (100) zu speichern;
b) Verarbeitungsmittel (62) zum Berechnen von Einstellparametern (130) als Funktion von Eigenschaften des Anfangsabschnitts (101) des Herzsignals (100).

6. Vorrichtung nach Anspruch 5, wobei die Lerneinheit (60) ferner einen Berechnungsblock (63) zum Berechnen der Hauptreferenzparameter (31) in Abhängigkeit von den Einstellparametern (130) umfasst.

## Revendications

1. Equipement pour détecter des signaux cardiaques, comprenant :
a) un moyen de capteur (10) pour détecter au moins un signal cardiaque (100) d'un patient ;
b) une unité de stockage (20) connectée audit moyen de capteur (10) pour stocker au moins partiellement ledit signal cardiaque (100) ;
c) une mémoire auxiliaire (30) contenant un ou plusieurs paramètres de référence principaux (31) pour ledit signal cardiaque (100) ;
d) un bloc comparateur (40) pour comparer ledit signal cardiaque (100) auxdits un ou plusieurs paramètres de référence principaux (31) et actionner ladite unité de stockage (20) en fonction de ladite comparaison au moyen d'un signal d'ordre principal (110) respectif ;
e) un bloc de pilotage (70) qui peut être actionné par le patient afin d'actionner ladite unité de stockage (20) au moyen d'un signal d'ordre auxiliaire (120) ;
f) un microphone (71), qui peut être actionné au moyen dudit bloc de pilotage (70) pour permettre audit patient d'entrer vocalement des premières informations (71a) concernant des symptômes perçus lors de l'actionnement dudit bloc de pilotage (70)
g) un afficheur à écran tactile (72), au moyen duquel ledit patient peut entrer des secondes informations (72a) concernant des symptômes perçus lors de l'actionnement dudit bloc de pilotage (70), dans lequel ledit afficheur (72) est configuré pour présenter audit patient une pluralité d'options à choisir afin d'entrer lesdites secondes informations (72a).

2. Equipement selon la revendication 1, dans lequel lesdites premières informations (71a) sont stockées dans une première zone de mémoire (71b) dudit équipement (1).

3. Equipement selon la revendication 1, dans lequel la possibilité d'entrer lesdites secondes informations (72b) au moyen dudit afficheur (72) est activée par ledit bloc de pilotage (70).

4. Equipement selon l'une quelconque des revendications précédentes, dans lequel lesdites secondes informations (72a) sont stockées dans une seconde zone de mémoire (72b) dudit équipement (1).

5. Equipement selon l'une quelconque des revendications précédentes, comprenant en outre une unité d'apprentissage (60) pourvue :
a) d'un registre d'apprentissage (61) pour stocker au moins une portion initiale (101) dudit signal cardiaque (100) ;
b) d'un moyen de traitement (62) pour calculer des paramètres de réglage (130) en fonction de caractéristiques de ladite portion initiale (101) du signal cardiaque (100).

6. Équipement selon la revendication 5, dans lequel ladite unité d'apprentissage (60) comprend en outre un bloc de calcul (63) pour calculer lesdits paramètres de référence principaux (31) en fonction desdits paramètres de réglage (130).
